# EUROPEAN PATENT SPECIFICATION

(11) **EP 0 852 224 B1**
(45) Date of publication and mention of the grant of the patent: **31.10.2001**
(21) Application number: 98300008.4
(22) Date of filing: 02.01.1998
(51) Int. Cl.: C07C 205/11, C07C 205/19, C07C 211/52, C07C 309/63, C07C 201/12, C07C 209/68, C07C 303/30, C07C 221/00, C07D 239/00, C07D 251/00

(54) **Aryne intermediates towards the preparation of herbicidal sulfamoyl urea and a process for their preparation**
Aryne-Zwischenverbindungen zur Herstellung von herbiziden Sulphamoylharnstoffen und ein Verfahren zu deren Herstellung
Intermédiaires arynes pour la préparation de sulphamoylurées herbicides et un procédé pour leur préparation

(30) Priority: 06.01.1997 US 34754 P; 04.02.1997 US 794961
(43) Date of publication of application: 08.07.1998
(73) Proprietor: American Cyanamid Company, Madison, New Jersey 07940-0874 (US)
(72) Inventor: Harrington, Philip Mark, Pennington, New Jersey 08534 (US); Kremer, Kenneth Alfred Martin, Lawrenceville, New Jersey 08648 (US)
(74) Representative: Langfinger, Klaus Dieter, Dr.

(56) References cited:
- EP-A- 0 380 257
- EP-A- 0 604 705
- GB-A- 1 527 783
- GB-A- 1 545 341
- GB-A- 2 281 296
- US-A- 3 562 277
- RUDISILL, D.E. AND STILLE, J.K.: "Palladium-catalysed synthesis of 2-substituted indoles" JOURNAL OF ORGANIC CHEMISTRY, vol. 54, no. 25, 1989, pages 5856-5866, XP002057066

## Description

Herbicidal sulfamoyl urea compounds are described in WO 95/29902, EP 661,276, WO 95/29167 and U.S. 4,622,065. A particularly potent and highly cereal selective sulfamoyl urea herbicidal agent is the subject of U. S. patent 5,009,699. This agent is especially useful for the selective control of a variety of weed species in the presence of cereal crops and particularly for controlling broadleaf weeds and sedges in the presence of transplanted or paddy rice. Therefore, alternate, effective, cost-efficient methods of manufacture of herbicidal sulfamoyl ureas, and particularly cereal-selective sulfamoyl ureas, are of continued interest.

Certain key intermediates in the manufacture of the above-said herbicides and the cereal-selective herbicide, 1-{[o-(cyclopropylcarbonyl)phenyl]sulfamoyl}-3-(4,6-dimethoxy-2-pyrimidinyl)urea, such as o-aminophenyl cyclopropyl ketone, o-nitrophenyl cyclopropyl ketone, 4-halo-o-nitrobutyrophenone, and 4-halo-o-aminobutyrophenone are described in U.S. Patents 5,362,911; 5,364,968; 5,405,998 and 5,414,136. Other preparative methods are described, for instance, in GB 1,527,783 which details the preparation of 4-halo-o-nitrobutyrophenone via the coupling of an o-nitrobenzoic acid halide with an α-alkanoyl-γ-butyrolactone in a three-step process; GB 1,545,341 which shows the synthesis of 4-chloro-o-amino-m-halo-butyrophenones via reaction of an aniline unsubstituted in at least one ortho position with a nitrile in the presence of boron trihalogenide to introduce a substituted iminomethyl into the ortho position of said aniline and hydrolyzing the resulting iminomethyl compound; GB 2,281,296 which details the preparation of 2-(4-chlorobutyryl)-4-chloroaniline via condensation of 4-chloroaniline with 4-chlorobutyronitrile, boron trichloride and an auxiliary Lewis acid; EP 0604705 which shows the preparation of 4-halo-o-nitrobutyrophenone using a mixture of 4-hydroxy-o-nitrobutyrophenone and tetrahydro-2-(o-nitrophenyl)-2-furanol and concentrated hydrochloric acid at reflux temperatures; EP 0380257 which shows the reaction of aniline and 4-chlorobutyronitrile in the presence of boron trichloride and aluminum chloride at reflux temperatures for 20 hours to prepare 1-(2-aminophenyl)-4-chlorobutan-1-one hydrochloride; and U. S. Patent 3,562,277 which details the preparation of 4-halo-o-nitrobutyrophenones via treatment of a 4-halobutyrophenone with nitric acid in the presence of an acid solvent, followed by fractional crystallization or chromatography to separate the desired product.

Rudisill *et al.* J. Org. Chem., 1989 Vol. 54, pages 5856-5866, disclose the preparation of (t-butyldimethylsilyl)oxy-2-alkynylanilines which are useful in the synthesis of phenylindoles. These intermediates are obviously not useful in the process for the preparation of 1-{o -(cyclopropylcarbonyl) phenyl] sulfamoyl}-3-(4,6-dimethoxy-2-pyrimidinyl)urea since such a process requires the ring formation at the terminal end of the alkyl chain rather than the formation of the heteroaryl phenylindole ring structure.

However, alternate and effective methods to prepare these key intermediate alkyl and cycloalkyl phenyl ketones and their derivatives from readily available, non-toxic starting materials are still being sought.

### SUMMARY OF THE INVENTION

The present invention provides aryne compounds of formula I 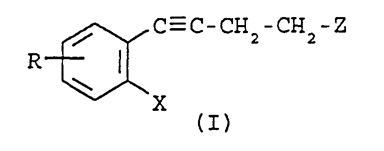 wherein
R is H, CN, F, formyl, C₁-C₄alkyl optionally substituted with one or more halogen, C₁-C₃alkoxy, C₁-C₃alkylthio, C₁-C₃alkylsulfinyl or C₁-C₃alkylsulfonyl groups,
C₁-C₄alkoxy optionally substituted with one or more halogen, C₁-C₃alkoxy, C₁-C₃alkylthio, C₁-C₃-alkylsufinyl or C₁-C₃alkylsulfonyl groups,
C₁-C₄alkylthio optionally substituted with one or more halogen, C₁-C₃alkoxy, C₁-C₃alkylthio, C₁-C₃-alkylsufinyl or C₁-C₃alkylsulfonyl groups,
C₁-C₄alkylsulfinyl optionally substituted with one or more halogen, C₁-C₃alkoxy, C₁-C₃alkylthio, C₁-C₃-alkylsufinyl or C₁-C₃alkylsulfonyl groups,
C₁-C₄alkylsulfonyl optionally substituted with one or more halogen, C₁-C₃alkoxy, C₁-C₃alkylthio, C₁-C₃-alkylsufinyl or C₁-C₃alkylsulfonyl groups,
C₁-C₄alkylcarbonyl optionally substituted with one or more halogen, C₁-C₃alkoxy, C₁-C₃alkylthio, C₁-C₃-alkylsufinyl or C₁-C₃alkylsulfonyl groups,
C₁-C₄alkoxycarbonyl optionally substituted by one or more halogen or C₁-C₃alkoxy groups,
di(C₁-C₄alkyl)amino optionally substituted by one or more halogen or C₁-C₃alkoxy groups,
di(C₁-C₄alkyl)aminocarbonyl optionally substituted by one or more halogen or C₁-C₃alkoxy groups,
di(C₁-C₄alkyl)aminosulfonyl optionally substituted by one or more halogen or C₁-C₃alkoxy groups, or
a heterocyclic ring having 2 to 6 carbon atoms and 1 to 3 nitrogen, oxygen or sulfur atoms and being optionally substituted on the carbon atoms with one or more fluorine, C₁-C₄alkyl or C₁-C₄haloalkyl groups;
X is NO₂ or NR₁R₂ ;
Z is OH, Br, Cl or OSO₂R₃;

- R₁ and R₂: are each independently hydrogen, formyl, acetyl, haloacetyl, CO₂R₄ or benzyl optionally substituted on the aromatic ring with one to three fluorine, nitro, C₁-C₃alkyl or C₁-C₃alkoxy groups; and
- R₃ and R₄: are each independently C₁-C₆alkyl, C₁-C₆haloalkyl or phenyl optionally substituted with one to three fluorine, nitro, C₁-C₃alkyl or C₁-C₃alkoxy groups; or
when X is NR₁R₂, the acid addition salts thereof.

Also provided is a method to prepare the formula I compounds via the palladium catalyzed coupling of an o-halonitrobenzene or o-haloaniline with 3-butyne-1-ol.

The formula I aryne compounds are useful as intermediates in the manufacture of sulfamoyl urea herbicidal agents.

### DETAILED DESCRIPTION OF THE INVENTION

Sulfamoyl urea derivatives are useful as herbicidal agents, in particular, 1-{[o-(cyclopropylcarbonyl)phenyl]sulfamoyl}-3-(4,6-dimethoxy-2-pyrimidinyl)urea is a potent, environmentally benign herbicide with important cereal crop selectivity. A key intermediate in the manufacture of this particular herbicide is 1-(o-anilino)-4-halo-1-butanone. This key intermediate may be obtained from the corresponding o-nitrobenzene precursor or alternatively from the 4-hydroxy-anilino- or 4-hydroxy-nitrobenzene-1-butanone precursors.

It has now been found that aryne compounds of formula I 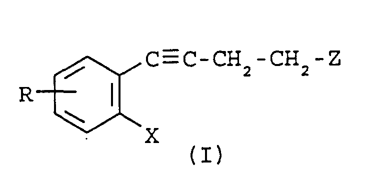 wherein
R is H, NH₂, CN, NO₂, F, formyl, C₁-C₄alkyl optionally substituted with one or more halogen, C₁-C₃alkoxy, C₁-C₃alkylthio, C₁-C₃alkylsulfinyl or C₁-C₃alkylsulfonyl groups,
C₁-C₄alkoxy optionally substituted with one or more halogen, C₁-C₃alkoxy, C₁-C₃alkylthio, C₁-C₃-alkylsufinyl or C₁-C₃alkylsulfonyl groups,
C₁-C₄alkylthio optionally substituted with one or more halogen, C₁-C₃alkoxy, C₁-C₃alkylthio, C₁-C₃-alkylsufinyl or C₁-C₃alkylsulfonyl groups,
C₁-C₄alkylsulfinyl optionally substituted with one or more halogen, C₁-C₃alkoxy, C₁-C₃alkylthio, C₁-C₃-alkylsufinyl or C₁-C₃alkylsulfonyl groups,
C₁-C₄alkylsulfonyl optionally substituted with one or more halogen, C₁-C₃alkoxy, C₁-C₃alkylthio, C₁-C₃-alkylsufinyl or C₁-C₃alkylsulfonyl groups,
C₁-C₄alkylcarbonyl optionally substituted with one or more halogen, C₁-C₃alkoxy, C₁-C₃alkylthio, C₁-C₃-alkylsufinyl or C₁-C₃alkylsulfonyl groups,
C₁-C₄alkoxycarbonyl optionally substituted by one or more halogen or C₁-C₃alkoxy groups,
di(C₁-C₄alkyl)amino optionally substituted by one or more halogen or C₁-C₃alkoxy groups,
di(C₁-C₄alkyl)aminocarbonyl optionally substituted by one or more halogen or C₁-C₃alkoxy groups,
di(C₁-C₄alkyl)aminosulfonyl optionally substituted by one or more halogen or C₁-C₃alkoxy groups, or
a heterocyclic ring having 2 to 6 carbon atoms and 1 to 3 nitrogen, oxygen or sulfur atoms and being optionally substituted on the-carbon atoms with one or more fluorine, C₁-C₄alkyl or C₁-C₄haloalkyl groups;
X is NO₂ or NR₁R₂;
Z is OH, Br, Cl or OSO₂R₃;
R₁ and R₂ are each independently hydrogen, formyl, acetyl, haloacetyl, CO₂R₄ or benzyl optionally substituted on the aromatic ring with one to three fluorine, nitro, C₁-C₃alkyl or C₁-C₃alkoxy groups; and
R₃ and R₄ are each independently C₁-C₆alkyl, C₁-C₆haloalkyl or phenyl optionally substituted with one to three fluorine, nitro, C₁-C₃alkyl or C₁-C₃alkoxy groups; or
when X is NR₁R₂, the acid addition salts thereof are useful in the preparation of important 4-halo or 4-hydroxy-o-substituted benzene-butanone intermediates in the manufacture of sulfamoyl urea herbicides.

The term haloacetyl designates an acetyl group substituted by one to three halogen atoms which may be the same or different. The term halogen as used in this definition of haloacetyl designates Cl, Br, I or F.

Preferred compounds of formula I are those compounds wherein R is H; Z is OH; and R₁ and R₂ are each independently H.

Advantageously, the aryne compounds of formula I may be prepared from readily available, non-toxic starting materials such as o-halobenzenes of formula III, preferably o-iodobenzenes or o-bromobenzenes and alkynes of formula IV, preferably 3-butyne-1-ol. In accordance with the method of invention, a formula III o-halobenzene, preferably o-iodoaniline or o-bromoaniline, may be reacted with at least one molar equivalent of a formula IV alkyne, preferably 3-butyne-1-ol, in the presence of a catalytically effective amount of a palladium(II) catalyst and a copper(I) catalyst and in the presence of an organic amine. The reaction is illustrated in flow diagram I wherein Hal is I, Cl or Br and R, X and Z are defined hereinabove.

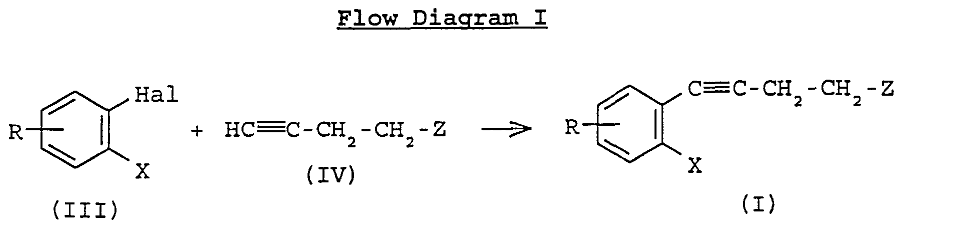

Organic amines suitable for use in the method of invention are any of those commonly used in manufacturing procedures such as triethylamine, diethylamine, tributylamine, diisopropylamine, and the like, preferably triethylamine.

Palladium catalysts may be any known catalyst wherein the palladium metal is present with a valence of two, such as bis(triphenylphosphine)palladium(II)chloride, palladium(II)acetate, palladium(II)chloride, and the like, preferably bis(triphenylphosphine)palladium(II)chloride. Similarly, the copper catalyst may be any known catalyst wherein the copper metal is present with a valence of one such as a copper(I)halide, preferably copper(I)iodide.

Conveniently the aryne compounds of the invention may be readily converted to key 4-halo or 4-hydroxy-o-substituted benzene-butanone intermediates essential in the manufacture of sulfamoyl urea herbicidal agents. In one embodiment of the invention, compounds of formula I wherein Z is hydroxy may be converted to the desired 4-hydroxyphenyl butanones of formula II in a single hydration step or, preferably, the desired formula II compound may be prepared in a single contiguous or continuous process.

In accordance with one embodiment of the inventive process, an o-halobenzene of formula III may be reacted with at least one molar equivalent of 3-butyne-1-ol in the presence of a catalytically effective amount of a palladium(II) catalyst and copper(I)catalyst and in the presence of an organic amine to give an aryne intermediate of formula Ia and the formula Ia intermediate may be hydrated with a hydrating agent and water, optionally at an elevated temperature, to give the desired formula II compound. The reaction sequence is shown in flow diagram II wherein Hal designates I, Cl or Br and R and X are as described hereinabove for formula I.

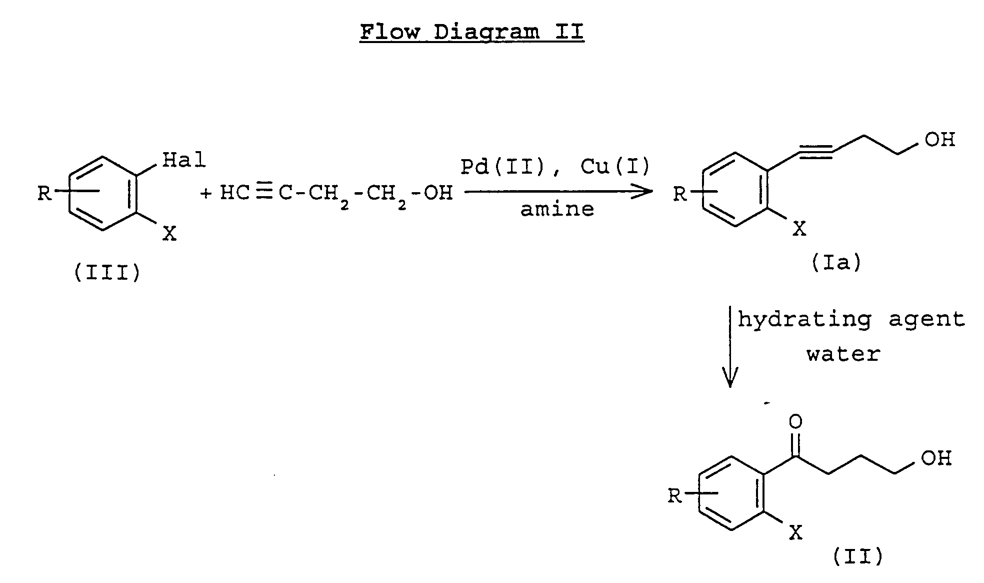

Hydrating agents suitable for use may be those commonly known in the art such as HgO, H₂SO₄; Na₂S, HCl; and the like.

Although the inventive process may be practiced at room temperature, it is understood that increased temperatures may decrease reaction time. However, excessively high temperatures may have adverse effects such as decomposition of reactants or products or facilitation of undesired side reactions. Suitable reaction temperatures range from about 0°C to 110°C, preferably about 25°C to 90°C.

The compound of formula II may be converted to the corresponding cyclopropyl ketone by displacing the hydroxy moiety with halogen, for example, by reacting the formula II butanone with a hydrogen halide to form the corresponding 4-halobutanone of formula V and dehydrohalogenating the resultant halobutanone to give the desired cyclopropyl product of formula VI. The reaction sequence is shown in flow diagram III.

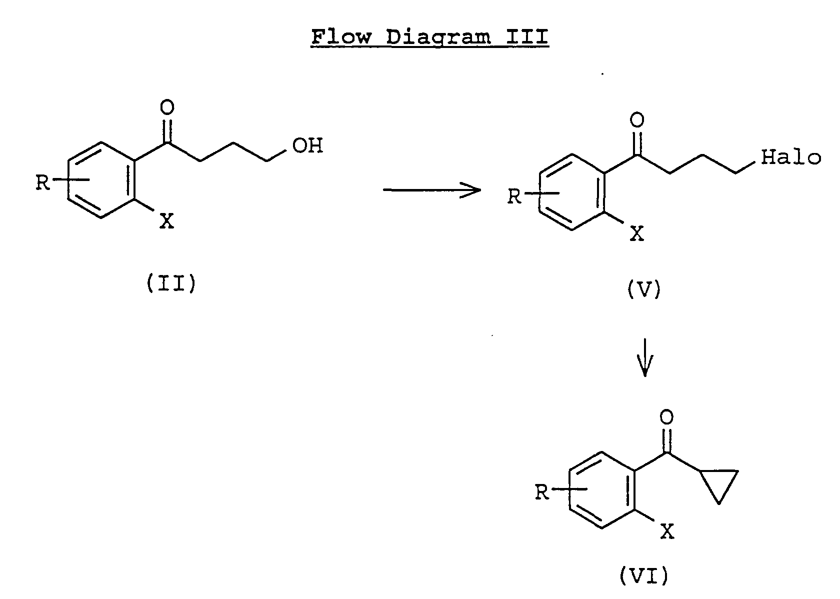

Methods to convert compounds of formula V wherein R is hydrogen to corresponding compounds of formula VI are described in U. S. 5,362,911. Methods to convert the formula VI cyclopropyl ketone wherein R is hydrogen and X is NH₂ to a cereal-selective, herbicidal, sulfamoyl urea are described in U. S. 5,008,699. Similarly, methods to convert the formula VI cyclopropyl ketone wherein R is hydrogen and X is NO₂ to a cereal-selective, herbicidal, sulfamoyl urea are described in U. S. 5,364,968. Methods to convert general phenyl ketones of formula V to their corresponding sulfamoyl urea herbicidal products are described in WO 95/29902, EP 661,276, WO 95/29167 and U.S. 4,622,065.

In accordance with another embodiment of the invention the halobenzene of formula III may be reacted with 3-butyne-1-ol in the presence of a catalytically effective amount of a palladium(II) catalyst and a copper(I) catalyst and in the presence of an organic amine to form the aryne intermediate of formula Ia. The formula Ia aryne may then be hydrated with hydrating agent to form the 4-hydroxyphenylbutanone of formula II. The formula II butanone may be halogenated with a hydrogen halide to give the corresponding 4-halo compound of formula V, which may then be dehydrohalogenated to give the cyclopropyl phenyl ketone of formula VI. When the formula VI compound is the compound wherein X is NH₂, said compound may be reacted with a 2-aminoaryl compound of formula VIII and chlorosulfonyl isocyanate in the presence of triethylamine and a solvent to give the desired herbicidal product of formula VII. The reaction sequence is shown in flow diagram IV wherein Halo designates Cl, Br, I or F; A is N or CR₆;
- R₅ is: H, halogen, C₁-C₄alkyl optionally substituted with one or more halogen or C₁-C₃alkoxy groups,

C₁-C₄alkoxy optionally substituted with one or more halogen or C₁-C₃alkoxy groups,
C₁-C₄alkylthio optionally substituted with one or more halogen or C₁-C₃alkoxy groups,
C₁-C₄alkylsulfinyl optionally substituted with one or more halogen or C₁-C₃alkoxy groups,
C₁-C₄alkylsulfonyl optionally substituted with one or more halogen or C₁-C₃alkoxy groups, or
C₁-C₄alkylamino or di (C₁-C₄alkyl)amino each alkyl group being optionally substituted with one or more halogen or C₁-C₃alkoxy groups;

- R₆ is: H or halogen; and
- R₇ is: hydrogen, C₁-C₄alkyl optionally substituted with one or more halogen or C₁-C₃alkoxy groups,

C₁-C₄alkoxy optionally substituted with one or more halogen or C₁-C₃alkoxy groups,
C₁-C₄alkylthio optionally substituted with one or more halogen or C₁-C₃alkoxy groups,
C₁-C₄alkylsulfinyl optionally substituted with one or more halogen or C₁-C₃alkoxy groups,
C₁-C₄alkylsulfonyl optionally substituted with one or more halogen or C₁-C₃alkoxy groups, or
C₁-C₄alkylamino or di(C₁-C₄alkyl)amino each alkyl group being optionally substituted with one or more halogen or C₁-C₃alkoxy group.

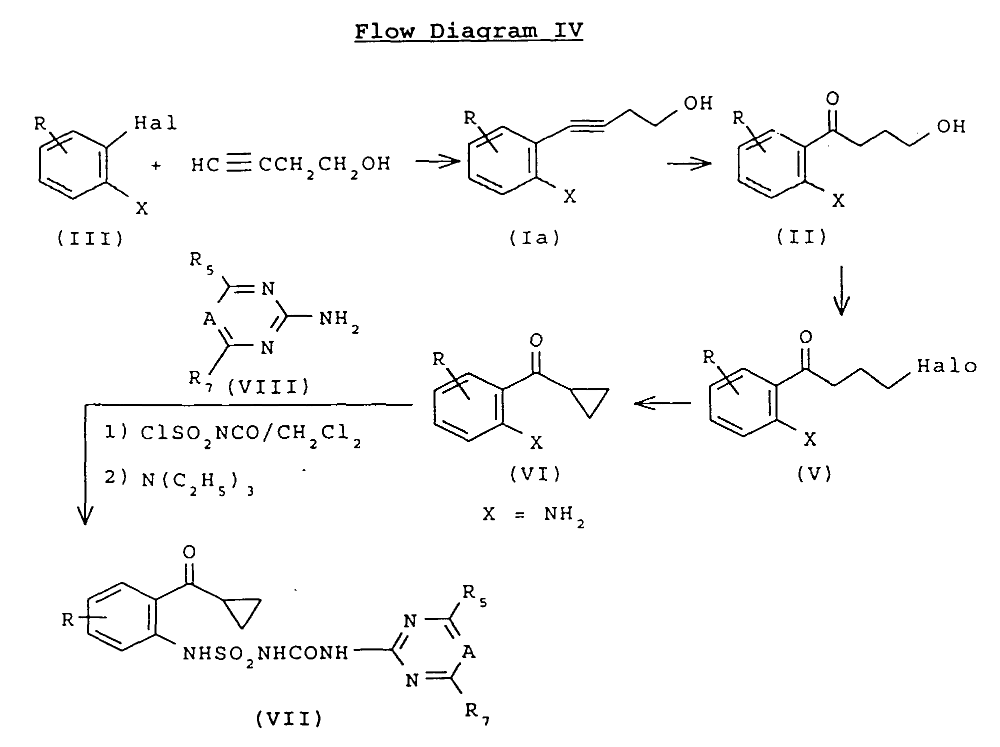

It is understood that compounds of formula II, V or VI wherein X is NO₂ or NR₁R₂ and either or both of R₁R₂ is other than hydrogen, may be converted to the corresponding aniline analogues by catalytic reduction or hydrolysis techniques, respectively. For example, when X is NO₂ for a compound of formula II, V or VI, said compound may be reduced with hydrogen gas in the presence of a catalyst optionally in the presence of a solvent to give the corresponding compound of formula II, V or VI wherein X is NH₂. Similarly, using known hydrolysis methods, a compound of formula II, V or VI wherein X is NR₁R₂ and either one or both of R₁ and R₂ are other than hydrogen may be hydrolyzed to give the corresponding compound of formula II, V or VI wherein X is NH₂.

The compound of formula VI where X is NH₂ and R is as defined above may therefore by prepared from other compounds of formula VI by reduction or hydrolysis to convert X into -NH₂. The compound of formula VI where X is NH₂ may be converted into a compound having formula VII by reaction with sulfonamide-forming reagent that introduces a group having formula X 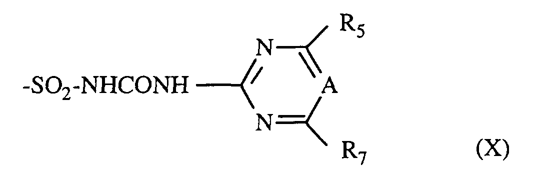 where A, R₅ and R₇ are as defined above.

For a more clear understanding of the invention, the following examples are set forth below. The term NMR designates nuclear magnetic resonance.

### EXAMPLE 1

### Preparation of 4-(o-anilino)-3-butyne-1-ol

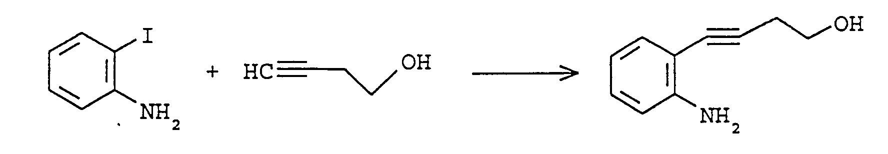

A solution of o-iodoaniline (35.0 g, 0.16 mol) in 640 ml of triethylamine, under nitrogen, is treated sequentially with bis(triphenylphosphine)palladium(II)chloride (2.24 g, 0.0032 mol), 3-butyne-1-ol (16.8 g, 0.24 mol) and copper(I)iodide (0.3 g, 0.0016 mol), stirred for 4.5 hours and filtered. The filtercake is washed with toluene and the filtrates are combined and concentrated *in vacuo* to give a dark oil residue. The residue is dissolved in methylene chloride, washed with water and filtered through a silica gel pad. The filtrate is concentrated *in vacuo* to give the title product as a dark oil, 25.2 g, 98% yield, identified by NMR and mass spectral analyses.

### EXAMPLE 2

### Preparation of 4-(o-nitrophenyl)-3-butyne-1-ol

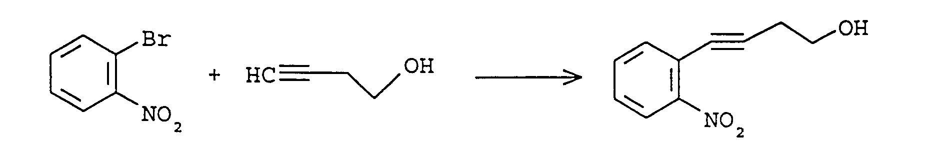

A solution of o-bromonitrobenzene (8.1 g, 0.04 mol) in 160 ml of triethylamine, under nitrogen, is treated sequentially with bis (triphenylphosphine)palladium (II)chloride (0.28 g, 0.4 mmol), 3-butyne-1-ol (2.81 g, 0.04 mol) and copper(I)iodide (0.15 g, 0.8 mmol), stirred for about 16 hours and filtered. The filtercake is washed with toluene. The filtrates are combined and concentrated *in vacuo* to give the title product as a dark oil, 6.0 g, 78.5% yield, identified by NMR and mass spectral analyses.

### EXAMPLE 3

### Preparation of 4-hydroxy-(o-anilino)-1-butanone

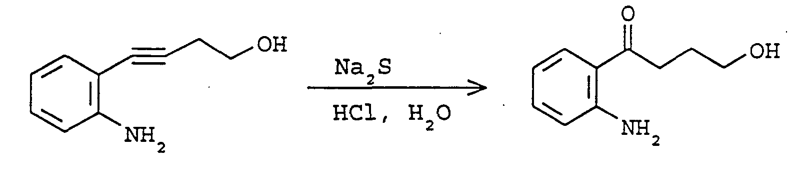

A solution of 4-(o-anilino)-3-butyne-1-ol (28 g, 90% pure, 0.155 mol) in 1.8 L methanol is treated sequentially with 1.35 kg of aqueous sodium sulfide (0.186 mol) and 176 g of dilute HCl (0.46 mol), heated at reflux for 9.4 hours, cooled to room temperature and filtered through celite. The filtrate is concentrated *in vacuo* and the concentrate is extracted with methylene chloride. The extracts are combined, washed with water and filtered through a silica gel pad. The filtrate is concentrated *in vacuo* to give the title product as a light orange solid, 16.6 g, 54.8% yield, identified by NMR and mass spectral analyses.

### EXAMPLE 4

### Preparation of 1-(o-anilino)-4-chloro-1-butanone hydrochloride

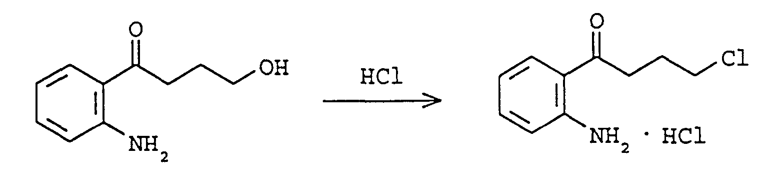

A mixture of 1-(o-anilino)-4-hydroxy-1-butanone (9.3 g, 5.1 mmol), 26 ml water and 90 ml of 37% HCl is heated at reflux temperature for 6.5 hours, cooled to room temperature and filtered. The filtercake is dried to give the title product as a white solid, 8.0 g. The filtrate is extracted with methylene chloride; the extracts are combined and the solvent evaporated *in vacuo* to give an additional 1.1 g of the title product, 73% overall yield, mp 142-145°C, identified by NMR and mass spectral analyses.

### EXAMPLE 5

### Preparation of o-aminophenyl cyclopropyl ketone

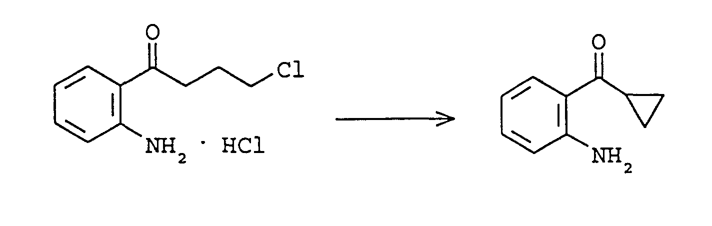

A solution of 1-(o-anilino)-4-chloro-1-butanone hydrochloride (0.3 g, 1.3 mmol) in a mixture of methylene chloride and ethylene dichloride is treated with 10% NaOH (1.2 g, 3.0 mmol) and 75% aqueous methyl tributylammonium chloride (0.05 g, 0.2 mmol), heated at 50°C for 5 hours and cooled to room temperature. The phases are separated and the aqueous phase is extracted with methylene chloride. The organic extracts are combined, washed with water and concentrated *in vacuo* to give the title product as a white solid, 0.14 g, 70% yield, mp 46-48°C, identified by NMR and mass spectral analyses.

### EXAMPLE 6

### Preparation of 1-(o-nitrophenyl)-4-hydroxy-1-butanone

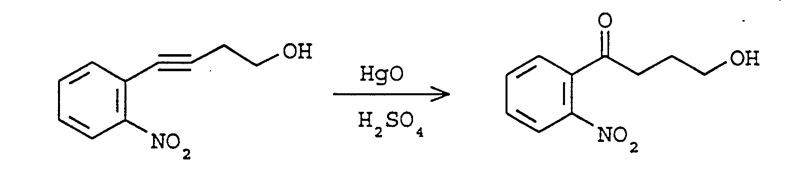

A mixture of HgO (0.21 g, 1.0 mmol) in 50 ml of 0.12N H₂SO₄ is heated to 60°C, treated dropwise with a solution of 4-(o-nitrophenyl)-3-butyne-1-ol (3.70 g, 19.4 mmol) in 25 ml of tetrahydrofuran over a 10 minute period, held at 60°C for 3 hours, treated with additional HgO (0.84 g, 3.9 mmol) suspended in 25 ml of 0.12N H₂SO₄, held at 60°C for 4 hours, cooled to room temperature and diluted with water. The resultant aqueous mixture is extracted with ether. The extracts are combined, washed with brine, dried over Na₂SO₄ and concentrated *in vacuo* to give a residue. The residue is purified by flash column chromatography (silica gel; gradient 1:1→2:1 ethyl acetate: hexanes as eluent) to give the title product as a brown oil, 1.48 g, 37% yield, identified by NMR and mass spectral analyses.

### EXAMPLE 7

### Preparation of 1-(o-nitrophenyl)-4-bromo-1-butanone

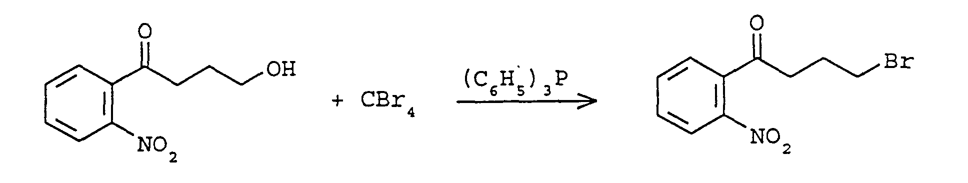

A mixture of 1-(o-nitrophenyl)-4-hydroxy-1-butanone (1.33 g, 6.4 mmol) and CBr₄ (2.32 g, 7.0 mmol) in toluene is treated with triphenylphospine (2.50 g, 9.5 mmol), heated at 60°C with stirring under nitrogen for 24 hours, cooled to room temperature and poured onto water. The resultant aqueous mixture is extracted with ethyl acetate. The extracts are combined, washed with brine, dried over Na₂SO₄ and concentrated *in vacuo* to give a residue. The residue is purified by flash column chromatography (silica gel; gradient 1:4→1:2 ethyl acetate: hexanes as eluent) to give the title product as a brown oil, 0.90 g, 52% yield, identified by NMR and mass spectral analyses.

### EXAMPLE 8

### Preparation of o-nitrophenyl cyclopropyl ketone

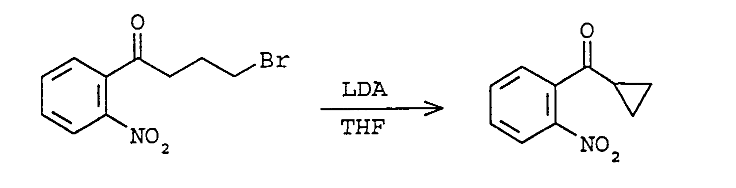

A solution of 1 (o-nitrophenyl)-4-bromo-1-butanone (0.75 g, 2.75 mmol) in dry tetrahydrofuran (THF) under nitrogen is cooled to -78°C with stirring, cautiously treated with 2.21 ml of 1.5 M lithium diisopropylamide (LDA) in cyclohexane (3.3 mmol LDA), held at -78°C for 1 hour, allowed to warm to 0°C for 2 hours and poured onto a saturated NH₄Cl solution. The resultant mixture is extracted with ethyl acetate. The extracts are combined, washed with brine, dried over Na₂SO₄ and concentrated *in vacuo* to give a residue. The residue is purified by flash column chromatography (silica gel; gradient 15%→25% ethyl acetate in hexane as eluent) to give the title product as a yellow oil, 0.34 g, 64% yield, identified by NMR and mass spectral analyses.

### EXAMPLE 9

### Preparation of 1-[[o-Cyclopropylcarbonyl)phenyl]sulfamoyl]-3-(4, 6-dimethoxy-2-pyrimidinyl) urea

The title compound is prepared by adding chlorosulfonyl isocyanate to an equimolar quantity of 2-amino-4, 6-dimethoxypyrimidine in dichloromethane at 0°C. 1-(Chlorosulfonyl) -3- (4, 6-dimethoxy-2-pyrimidinyl) urea is thereby formed in situ. o-Aminophenyl cyclopropyl ketone whose preparation is given in Example 5 and triethylamine are subsequently added.

One aspect of the invention resides in a process for the preparation of a compound having the formula VII as defined and illustrated above, which comprises
(a) hydrating a compound having the formula I as defined and illustrated above with a hydrating agent and, if Z is -OH, reacting the hydration product with a reagent for exchanging hydroxy by chlorine or bromine or with a reactive derivative of a sulfonic acid having the formula R₃SO₂OH where R₃ is as defined in above so as to convert the OH represented by Z into Br, Cl or OSO₂R₃ where R₃ is as defined above;
(b) cyclising the resultant compound having the formula IX 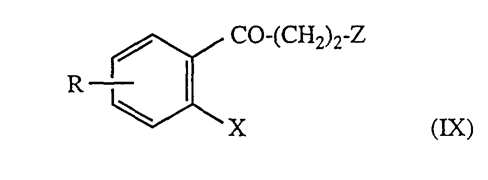 where R and X are as defined above and Z is Br, Cl or OSO₂R₃
   where R₃ is as defined in claim 1 so as to form a cyclopropylcarbonyl group at the position ortho to X and, if X is other than -NH₂ in the product obtained, converting X into -NH2 by reduction or hydrolysis; and
(c) reaction of the resultant compound having the formula VI as illustrated above wherein X is NH₂ and R is as defined above with a sulfonamide-forming reagent that introduces a group having the formula X 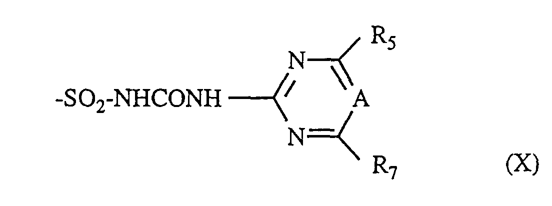 where A, R₅ and R₇ are as defined above.

## Claims

1. A compound of formula I 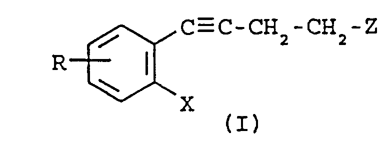 wherein
R is H, CN, F, formyl, C₁-C₄alkyl optionally substituted with one or more halogen, C₁C₃alkoxy, C₁-C₃alkylthio, C₁-C₃alkylsulfinyl or C₁-C₃alkylsulfonyl groups,
C₁-C₄alkoxy optionally substituted with one or more halogen, C₁-C₃alkoxy, C₁-C₃alkylthio, C₁-C₃-alkylsufinyl or C₁-C₃alkylsulfonyl groups,
C₁-C₄alkylthio optionally substituted with one or more halogen, C₁-C₃alkoxy, C₁-C₃alkylthio, C₁-C₃-alkylsufinyl or C₁-C₃alkylsulfonyl groups,
C₁-C₄alkylsulfinyl optionally substituted with one or more halogen, C₁-C₃alkoxy, C₁-C₃alkylthio, C₁-C₃-alkylsufinyl or C₁-C₃alkylsulfonyl groups,
C₁-C₄alkylsulfonyl optionally substituted with one or more halogen, C₁-C₃alkoxy, C₁-C₃alkylthio, C₁-C₃-alkylsufinyl or C₁-C₃alkylsulfonyl groups,
C₁-C₄alkylcarbonyl optionally substituted with one or more halogen, C₁-C₃alkoxy, C₁-C₃alkylthio, C₁-C₃-alkylsufinyl or C₁-C₃alkylsulfonyl groups,
C₁-C₄alkoxycarbonyl optionally substituted by one or more halogen or C₁-C₃alkoxy groups,
di(C₁-C₄alkyl)amino optionally substituted by one or more halogen or C₁-C₃alkoxy groups,
di(C₁-C₄alkyl)aminocarbonyl optionally substituted by one or more halogen or C₁-C₃alkoxy groups,
di(C₁-C₄alkyl)aminosulfonyl optionally substituted by one or more halogen or C₁-C₃alkoxy groups, or
a heterocyclic ring having 2 to 6 carbon atoms and 1 to 3 nitrogen, oxygen or sulfur atoms and being optionally substituted on the carbon atoms with one or more fluorine, C₁-C₄alkyl or C₁-C₄haloalkyl groups;
X is NO₂ or NR₁R₂;
Z is OH, Br, Cl or OSO₂R₃;
R₁ and R₂ are each independently hydrogen, formyl, acetyl, haloacetyl, CO₂R₄ or benzyl optionally substituted on the aromatic ring with one to three fluorine, nitro, C₁-C₃alkyl or C₁-C₃alkoxy groups; and
R₃ and R₄ are each independently C₁-C₆alkyl, C₁-C₆haloalkyl or phenyl optionally substituted with one to three fluorine, nitro, C₁-C₃alkyl or C₁-C₃alkoxy groups; or
when X is NR₁R₂, the acid addition salts thereof.

2. The compound according to claim 1 wherein R is H.

3. The compound according to claim 2 wherein X is NO₂ or NH₂.

4. The compound according to claim 2 or 3 wherein Z is OH.

5. A process for the preparation of a compound of formula I 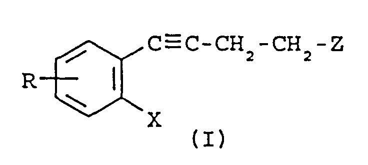 wherein
R is defined in claim 1;
X is NO₂ or NR₁R₂;
Z is OH, Br, Cl or OSO₂R₃;
R₁ and R₂ are each independently hydrogen, formyl, acetyl, haloacetyl, CO₂R₄ or benzyl optionally substituted on the aromatic ring with one to three fluorine, nitro, C₁-C₃alkyl or C₁-C₃alkoxy groups; and
R₃ and R₄ are each independently C₁-C₆alkyl, C₁-C₆haloalkyl or phenyl optionally substituted with one to three fluorine, nitro, C₁-C₃alkyl or C₁-C₃alkoxy groups
which comprises reacting a halobenzene of formula III 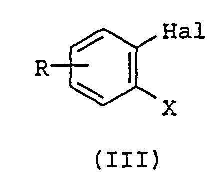 wherein Hal is Cl, I or Br and R and X are as defined for formula I with at least one molar equivalent of a butyne compound of formula IV
HC≡C-CH₂-CH₂-Z (IV)
wherein Z is as defined for formula I in the presence of a catalytically effective amount of a palladium(II)catalyst and a copper(I)catalyst and in the presence of an organic amine.

6. A process for the preparation of a compound of formula II 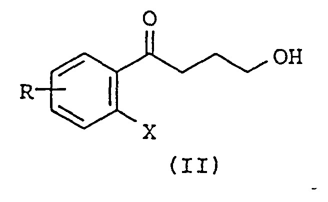
wherein R is defined in claim 1;
X is NO₂ or NR₁R₂;
R₁ and R₂ are each independently hydrogen, formyl, acetyl, haloacetyl, CO₂R₄ or benzyl optionally substituted on the aromatic ring with one to three fluorine, nitro, C₁-C₃alkyl or C₁-C₃alkoxy groups; and
R₄ is C₁-C₆alkyl, C₁-C₆haloalkyl or phenyl optionally substituted with one to three fluorine, nitro, C₁-C₃alkyl or C₁-C₃alkoxy groups
which comprises reacting a compound of formula III 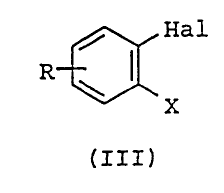 wherein X and R are as defined above and Hal is Cl, I or Br with at least one molar equivalent of 3-butyne-1-ol in the presence of a catalytically effective amount of a palladium(II)catalyst and a copper(I)catalyst and in the presence of an organic amine to give an intermediate of formula Ia 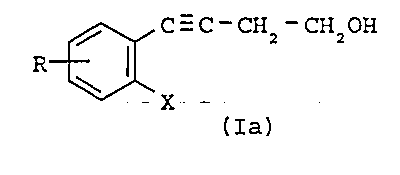 wherein R and X are defined above and hydrating the formula Ia intermediate in the presence of water and a hydrating agent to give the desired formula II compound.

7. A process for the preparation of a compound of formula VII 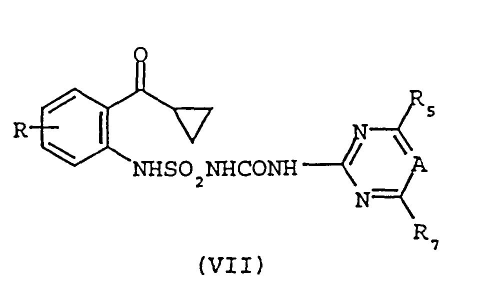 wherein
R is defined in claim 1 and
A is N or CR₆;
R₅ is H, halogen, C₁-C₄alkyl optionally substituted with one or more halogen or C₁-C₃alkoxy groups,
C₁-C₄alkoxy optionally substituted with one or more halogen or C₁-C₃alkoxy groups,
C₁-C₄alkylthio optionally substituted with one or more halogen or C₁-C₃alkoxy groups,
C₁-C₄alkylsulfinyl optionally substituted with one or more halogen or C₁-C₃alkoxy groups,
C₁-C₄alkylsulfonyl optionally substituted with one or more halogen or C₁-C₃alkoxy groups, or
C₁-C₄alkylamino or di(C₁-C₄alkyl)amino each alkyl group being optionally substituted with one or more halogen or C₁-C₃alkoxy groups;
R₆ is H or halogen; and
R₇ is hydrogen, C₁-C₄alkyl optionally substituted with one or more halogen or C₁-C₃alkoxy groups,
C₁-C₄alkoxy optionally substituted with one or more halogen or C₁-C₃alkoxy groups,
C₁-C₄alkylthio optionally substituted with one or more halogen or C₁-C₃alkoxy groups,
C₁-C₄alkylsulfinyl optionally substituted with one or more halogen or C₁-C₃alkoxy groups,
C₁-C₄alkylsulfonyl optionally substituted with one or more halogen or C₁-C₃alkoxy groups, or
C₁-C₄alkylamino or di(C₁-C₄alkyl)amino each alkyl group being optionally substituted with one or more halogen or C₁-C₄alkoxy group
which comprises the following steps:
a) reacting a halobenzene of formula III 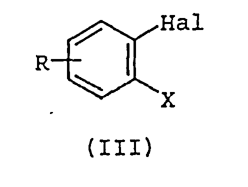 wherein R is defined in claim 1;
X is NO₂ or NH₂; and Hal is Cl, I or Br with at least one molar equivalent of 3-butyne-1-ol in the presence of a catalytically effective amount of a palladium(II)catalyst and a copper(I)catalyst and in the presence of an organic amine to form an aryne compound of formula Ia 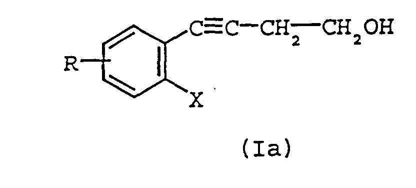 wherein R and X are defined hereinabove;
b) hydrating said formula I aryne with a hydrating agent to give a 4-hydroxy-(o-nitrophenyl)butanone or 4-hydroxy-o-aminophenyl)butanone of formula II 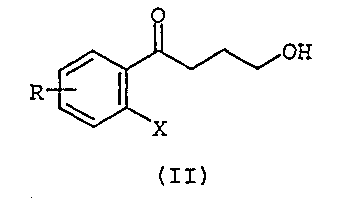 wherein R and X are defined hereinabove;
c) halogenating said 4-hydroxy-butanone with a hydrogen halide to give a 4-halo-butanone compound of formula V 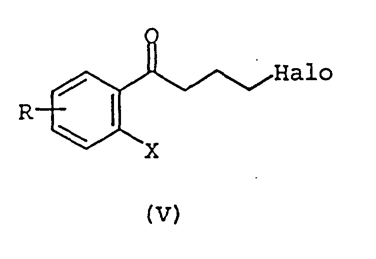 wherein R is defined above;
X is NO₂ or NH₂, and Halo is Cl, Br, I or F;
d) dehydrohalogenating said formula V compound in the presence of a base and a phase transfer catalyst and optionally in the presence of a solvent to form an o-nitrophenyl cyclopropyl ketone or o-aminophenyl cyclopropyl ketone of formula VI 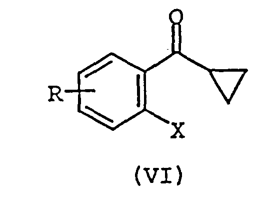 wherein R is defined above and X is NH₂ or NO₂;
e)when X is NO_{2,} reducing said o-nitrophenyl cyclopropyl ketone of formula VI in the presence of hydrogen and a catalyst optionally in the presence of a solvent to form the corresponding o-aminophenyl cyclopropyl ketone of formula VI wherein X is NH₂; and
f) reacting said formula VI o-aminophenyl cyclopropyl ketone with a 2-aminoaryl compound of formula VIII 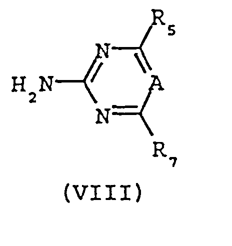 wherein A, R₅ and R₇ are defined above
and chlorosulfonyl isocyanate in the presence of triethylamine and a solvent to give the desired sulfamoyl urea compound of formula VII.

8. The process according to claim 5, 6, or 7 wherein the palladium(II)catalyst is bis(triphenylphosphine)-palladium(II)chloride and the copper(I)catalyst is copper(I)iodide; the organic amine is triethylamine or diethylamine; the process employs a formula III compound wherein R is H and X is NO₂ or NH₂.

9. The process according to claim 6 or 7 wherein the hydrating agent is HgO, H₂SO₄ or Na₂S, HCl.

10. The process according to claim 7 for preparing a compound of formula VII wherein A is CR₆; R and R₆ are each H; and R₅ and R₇ are each methoxy.

11. A process for the preparation of a compound having the formula VII as defined and illustrated in claim 7, which comprises
(a) hydrating a compound having the formula I as defined and illustrated in claim 1 with a hydrating agent and, if Z is -OH, reacting the hydration product with a reagent for exchanging hydroxy by chlorine or bromine or with a reactive derivative of a sulfonic acid having the formula R₃SO₂OH where R₃ is as defined in claim 1 so as to convert the OH represented by Z into Br, Cl or OSO₂R₃ where R₃ is as defined above;
(b) cyclising the resultant compound having the formula IX 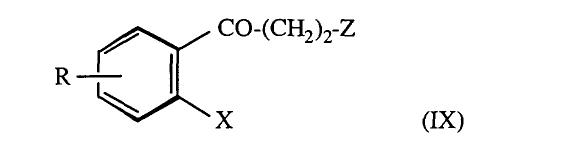 where R and X are as defined in claim 1 and Z is Br, Cl or OSO₂R₃ where R₃ is as defined in claim 1 so as to form a cyclopropylcarbonyl group at the position ortho to X and, if X is other than -NH₂ in the product obtained, converting X into -NH₂ by reduction or hydrolysis; and
(c) reaction of the resultant compound having the formula VI as illustrated in claim 7 wherein X is NH₂ and R is as defined in claim 1 with a sulfonamide-forming reagent that introduces a group having the formula X 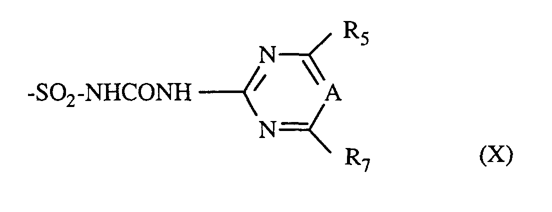 where A, R₅ and R₇ are as defined in claim 7.

## Patentansprüche

1. Verbindungen der Formel I 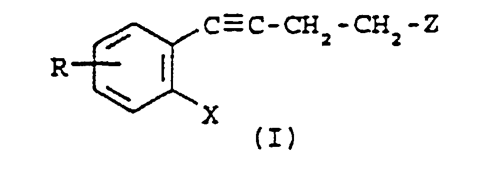
in welcher R für H, CN, F, Formyl, gegebenenfalls durch eine oder mehrere Halogen-, C₁-C₃-Alkoxy-, C₁-C₃-Alkylthio-, C₁-C₃-Alkylsulfinyl- oder C₁-C₃-Alkylsulfonylgruppen substituiertes C₁-C₄-Alkyl,
gegebenenfalls durch eine oder mehrere Halogen-, C₁-C₃-Alkoxy-, C₁-C₃-Alkylthio-, C₁-C₃-Alkylsulfinyl- oder C₁-C₃-Alkylsulfonylgruppen substituiertes C₁-C₄-Alkoxy,
gegebenenfalls durch eine oder mehrere Halogen-, C₁-C₃-Alkoxy-, C₁-C₃-Alkylthio-, C₁-C₃-Alkylsulfinyl- oder C₁-C₃-Alkylsulfonylgruppen substituiertes C₁-C₄-Alkylthio,
gegebenenfalls durch eine oder mehrere Halogen-, C₁-C₃-Alkoxy-, C₁-C₃-Alkylthio-, C₁-C₃-Alkylsulfinyl- oder C₁-C₃-Alkylsulfonylgruppen substituiertes C₁-C₄-Alkylsulfinyl,
gegebenenfalls durch eine oder mehrere Halogen-, C₁-C₃-Alkoxy-, C₁-C₃-Alkylthio-, C₁-C₃-Alkylsulfinyl- oder C₁-C₃-Alkylsulfonylgruppen substituiertes C₁-C₄-Alkylsulfonyl,
gegebenenfalls durch eine oder mehrere Halogen-, C₁-C₃-Alkoxy-, C₁-C₃-Alkylthio-, C₁-C₃-Alkylsulfinyl- oder C₁-C₃-Alkylsulfonylgruppen substituiertes C₁-C₄-Alkylcarbonyl,
gegebenenfalls durch eine oder mehrere Halogen- oder C₁-C₃-Alkoxygruppen substituiertes C₁-C₄-Alkoxycarbonyl,
gegebenenfalls durch eine oder mehrere Halogen- oder C₁-C₃-Alkoxygruppen substituiertes Di(C₁-C₄-alkyl)amino,
gegebenenfalls durch eine oder mehrere Halogen- oder C₁-C₃-Alkoxygruppen substituiertes Di(C₁-C₄-alkyl)aminocarbonyl,
gegebenenfalls durch eine oder mehrere Halogen- oder C₁-C₃-Alkoxygruppen substituiertes Di(C₁-C₄-alkyl)aminosulfonyl oder
einen heterocyclischen Ring mit 2 bis 6 Kohlenstoffatomen und 1 bis 3 Stickstoff-, Sauerstoff- oder Schwefelatomen steht, der an den Kohlenstoffatomen gegebenenfalls durch eine oder mehrere Fluor-, C₁-C₄-Alkyl- oder C₁-C₄-Halogenalkylgruppen substituiert ist;
X für NO₂ oder NR₁R₂ steht;
Z für OH, Br, Cl oder OSO₂R₃ steht;
R₁ und R₂ jeweils unabhängig voneinander für Wasserstoff, Formyl, Acetyl, Halogenacetyl, CO₂R₄ oder am aromatischen Ring gegebenenfalls durch eine bis drei Fluor-, Nitro-, C₁-C₃-Alkyl- oder C₁-C₃-Alkoxygruppen substituiertes Benzyl stehen; und
R₃ und R₄ jeweils unabhängig voneinander für C₁-C₆-Alkyl, C₁-C₆-Halogenalkyl oder gegebenenfalls durch eine bis drei Fluor-, Nitro-, C₁-C₃-Alkyl- oder C₁-C₃-Alkoxygruppen substituiertes Phenyl stehen; und,
falls X für NR₁R₂ steht, deren Säureadditionssalze.

2. Verbindungen nach Anspruch 1, in denen R für H steht.

3. Verbindungen nach Anspruch 2, in denen X für NO₂ oder NH₂ steht.

4. Verbindungen nach Anspruch 2 oder 3, in denen Z für OH steht.

5. Verfahren zur Herstellung von Verbindungen der Formel I 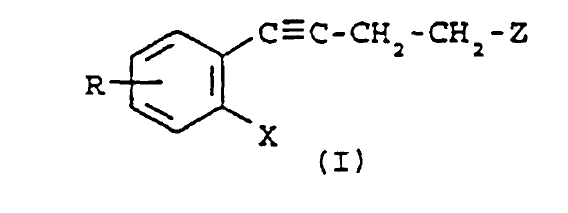
in welcher R wie in Anspruch 1 definiert ist;
X für NO₂ oder NR₁R₂ steht;
Z für OH, Br, Cl oder OSO₂R₃ steht;
R₁ und R₂ jeweils unabhängig voneinander für Wasserstoff, Formyl, Acetyl, Halogenacetyl, CO₂R₄ oder am aromatischen Ring gegebenenfalls durch eine bis drei Fluor-, Nitro-, C₁-C₃-Alkyl- oder C₁-C₃-Alkoxygruppen substituiertes Benzyl stehen; und
R₃ und R₄ jeweils unabhängig voneinander für C₁-C₆-Alkyl, C₁-C₆-Halogenalkyl oder gegebenenfalls durch eine bis drei Fluor-, Nitro-, C₁-C₃-Alkyl- oder C₁-C₃-Alkoxygruppen substituiertes Phenyl stehen,
**dadurch gekennzeichnet, daß** man ein Halogenbenzol der Formel III 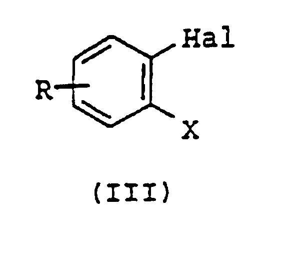 in welcher Hal für Cl, I oder Br steht und R und X wie unter Formel I definiert sind, mit wenigstens einem Moläquivalent einer Butinverbindung der Formel IV
HC≡C-CH₂-CH₂-Z (IV)
in welcher Z wie unter Formel I definiert ist, in Gegenwart einer katalytisch wirksamen Menge eines Palladium(II)-Katalysators und eines Kupfer(I)Katalysators und in Gegenwart eines organischen Amins umsetzt.

6. Verfahren zur Herstellung von Verbindungen der Formel II 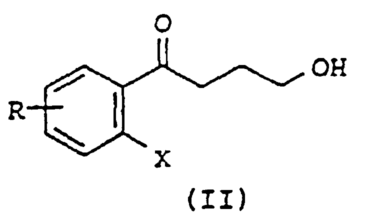
in welcher R wie in Anspruch 1 definiert ist;
X für NO₂ oder NR₁R₂ steht;
R₁ und R₂ jeweils unabhängig voneinander für Wasserstoff, Formyl, Acetyl, Halogenacetyl, CO₂R₄ oder am aromatischen Ring gegebenenfalls durch eine bis drei Fluor-, Nitro-, C₁-C₃-Alkyl- oder C₁-C₃-Alkoxygruppen substituiertes Benzyl stehen; und
R₄ für C₁-C₆-Alkyl, C₁-C₆-Halogenalkyl oder gegebenenfalls durch eine bis drei Fluor-, Nitro-, C₁-C₃-Alkyl- oder C₁-C₃-Alkoxygruppen substituiertes Phenyl steht,
**dadurch gekennzeichnet, daß** man eine Verbindung der Formel III 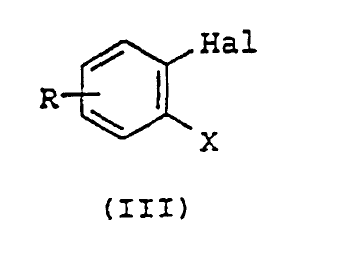 in welcher X und R wie oben definiert sind und Hal für Cl, I oder Br steht, mit wenigstens einem Moläquivalent But-3-in-1-ol in Gegenwart einer katalytisch wirksamen Menge eines Palladium(II)Katalysators und eines Kupfer(I)-Katalysators und in Gegenwart eines organischen Amins zu einem Zwischenprodukt der Formel Ia 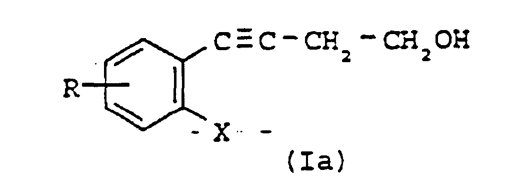 in welcher R und X wie oben definiert sind, umsetzt und das Zwischenprodukt der Formel Ia in Gegenwart von Wasser und einem Hydratisierungsmittel zur gewünschten Verbindung der Formel II hydratisiert.

7. Verfahren zur Herstellung einer Verbindung der Formel VII 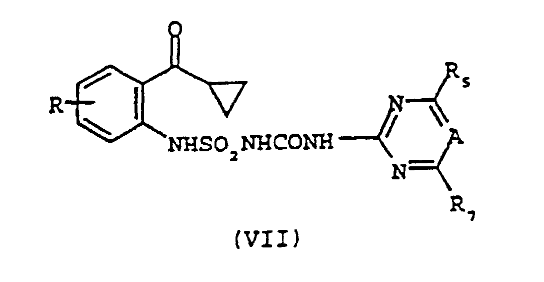
in welcher R wie in Anspruch 1 definiert ist und
A für N oder CR₆ steht;
R₅ für H, Halogen, gegebenenfalls durch eine oder mehrere Halogen- oder C₁-C₃-Alkoxygruppen substituiertes C₁-C₄-Alkyl,
gegebenenfalls durch eine oder mehrere Halogen- oder C₁-C₃-Alkoxygruppen substituiertes C₁-C₄-Alkoxy,
gegebenenfalls durch eine oder mehrere Halogen- oder C₁-C₃-Alkoxygruppen substituiertes C₁-C₄-Alkylthio,
gegebenenfalls durch eine oder mehrere Halogen- oder C₁-C₃-Alkoxygruppen substituiertes C₁-C₄-Alkylsulfinyl,
gegebenenfalls durch eine oder mehrere Halogen- oder C₁-C₃-Alkoxygruppen substituiertes C₁-C₄-Alkylsulfonyl, oder
C₁-C₄-Alkylamino oder Di (C₁-C₄-alkyl)amino steht, wobei die Alkylgruppen jeweils gegebenenfalls durch eine oder mehrere Halogen- oder C₁-C₃-Alkoxygruppen substituiert sind;
R₆ für H oder Halogen steht; und
R₇ für Wasserstoff, gegebenenfalls durch eine oder mehrere Halogen- oder C₁-C₃-Alkoxygruppen substituiertes C₁-C₄-Alkyl,
gegebenenfalls durch eine oder mehrere Halogen- oder C₁-C₃-Alkoxygruppen substituiertes C₁-C₄-Alkoxy,
gegebenenfalls durch eine oder mehrere Halogen- oder C₁-C₃-Alkoxygruppen substituiertes C₁-C₄-Alkylthio,
gegebenenfalls durch eine oder mehrere Halogen- oder C₁-C₃-Alkoxygruppen substituiertes C₁-C₄-Alkylsulfinyl,
gegebenenfalls durch eine oder mehrere Halogen- oder C₁-C₃-Alkoxygruppen substituiertes C₁-C₄-Alkylsulfonyl, oder
C₁-C₄-Alkylamino oder Di(C₁-C₄-alkyl)amino steht, wobei die Alkylgruppen jeweils gegebenenfalls durch eine oder mehrere Halogen- oder C₁-C₃-Alkoxygruppen substituiert sind,
**dadurch gekennzeichnet, daß** es die folgenden Schritte umfaßt:
a) Umsetzung eines Halogenbenzols der Formel III 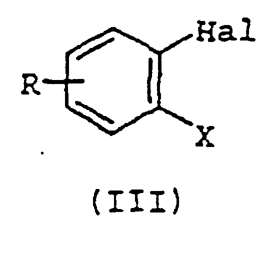 in welcher R wie in Anspruch 1 definiert ist;
X für NO₂ oder NH₂ steht und Hal für Cl, I oder Br steht, mit wenigstens einem Moläquivalent But-3-in-1-ol in Gegenwart einer katalytisch wirksamen Menge eines Palladium(II)-Katalysators und eines Kupfer(I)-Katalysators und in Gegenwart eines organischen Amins zu einer Arinverbindung der Formel Ia 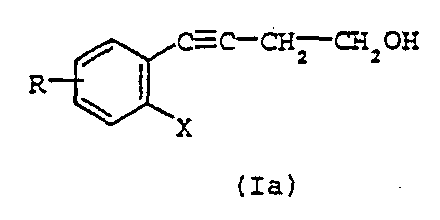 in welcher R und X wie oben definiert sind;
b) Hydratisierung des Arins der Formel I mit einem Hydratisierungsmittel zum 4-Hydroxy-(o-nitrophenyl)butanon bzw. 4-Hydroxy-(o-aminophenyl)butanon der Formel II 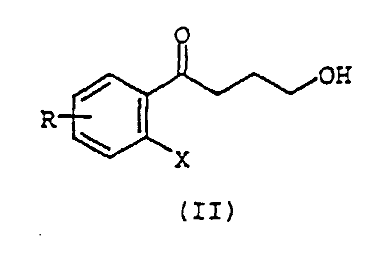 in welcher R und X wie oben definiert sind;
c) Halogenierung des 4-Hydroxybutanons mit einem Wasserstoffhalogenid zur 4-Halogenbutanonverbindung der Formel V 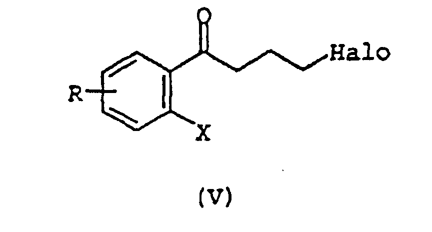 in welcher R wie oben definiert ist;
X für NO₂ oder NH₂ steht und Halo für Cl, Br, I oder F steht;
d) Dehydrohalogenierung der Verbindung der Formel V in Gegenwart einer Base und eines Phasentransferkatalysators und gegebenenfalls in Gegenwart eines Lösungsmittels zum o-Nitrophenylcyclopropyl-keton bzw. o-Aminophenyl-cyclopropylketon der Formel VI 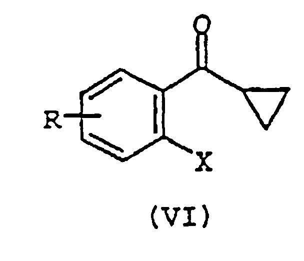 in welcher R wie oben definiert ist und X für NH₂ oder NO₂ steht;
e) in dem Fall, daß X für NO₂ steht, Reduktion des o-Nitrophenyl-cyclopropyl-ketons der Formel VI in Gegenwart von Wasserstoff und eines Katalysators und gegebenenfalls in Gegenwart eines Lösungsmittels zum entsprechenden o-Aminophenylcyclopropyl-keton der Formel VI, in der X für NH₂ steht; und
f) Umsetzung des o-Aminophenyl-cyclopropyl-ketons der Formel VI mit einer 2-Aminoarylverbindung der Formel VIII 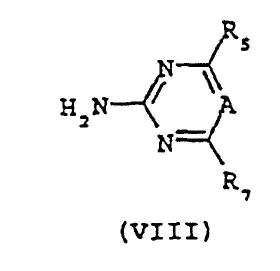 in welcher A, R₅ und R₇ wie oben definiert sind, und Chlorsulfonylisocyanat in Gegenwart von Triethylamin und einem Lösungsmittel zur gewünschten Sulfamoylharnstoffverbindung der Formel VII.

8. Verfahren nach Anspruch 5, 6 oder 7, bei dem es sich bei dem Palladium(II)-Katalysator um Bis(triphenylphosphin)palladium(II)-chlorid und beim Kupfer(I)-Katalysator um Kupfer(I)-iodid handelt; es sich bei dem organischen Amin um Triethylamin oder Diethylamin handelt und beim Verfahren eine Verbindung der Formel III eingesetzt wird, in welcher R für H und X für NO₂ oder NH₂ steht.

9. Verfahren nach Anspruch 6 oder 7, bei dem es sich bei dem Hydratisierungsmittel um HgO, H₂SO₄ oder Na₂S, HCl handelt.

10. Verfahren nach Anspruch 7 zur Herstellung einer Verbindung der Formel VII, in welcher A für CR₆ steht, R und R₆ jeweils für H und R₅ und R₇ jeweils für Methoxy stehen.

11. Verfahren zur Herstellung einer Verbindung der Formel VII, wie in Anspruch 7 definiert und erläutert, **dadurch gekennzeichnet, daß** man
(a) eine Verbindung der Formel I, wie in Anspruch 1 definiert und erläutert, mit einem Hydratisierungsmittel hydratisiert und, falls Z für -OH steht, das Hydratisierungsprodukt mit einem Reagens zum Austausch von Hydroxy gegen Chlor oder Brom oder mit einem reaktiven Derivat einer Sulfonsäure der Formel R₃SO₂OH, wobei R₃ wie in Anspruch 1 definiert ist, so umsetzt, daß die durch Z dargestellte OH-Gruppe in Br, Cl bzw. OSO₂R₃ umgewandelt wird, wobei R₃ wie oben definiert ist;
(b) die so erhaltene Verbindung der Formel IX 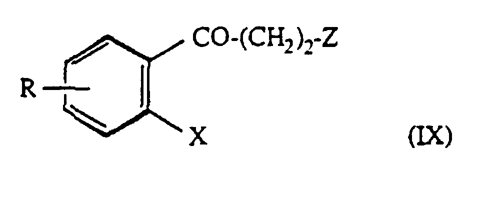 wobei R und X wie in Anspruch 1 definiert sind und Z für Br, Cl oder OSO₂R₃ steht, wobei R₃ wie in Anspruch 1 definiert ist, so cyclisiert, daß eine Cyclopropylcarbonylgruppe in der Position ortho zu X gebildet wird, und, wenn X in dem erhaltenen Produkt nicht für -NH₂ steht, X durch Reduktion oder Hydrolyse in -NH₂ umwandelt; und
(c) die so erhaltene Verbindung der Formel VI, wie in Anspruch 7 erläutert, in welcher X für NH₂ steht und R wie in Anspruch 1 definiert ist, mit einem Sulfonamid bildenden Reagens, das eine Gruppe der Formel X 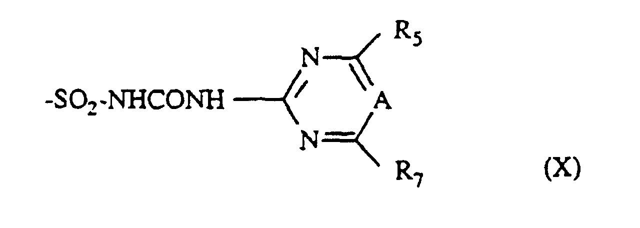 einführt, wobei A, R₅ und R₇ wie in Anspruch 7 definiert sind, umsetzt.

## Revendications

1. Composé de formule I 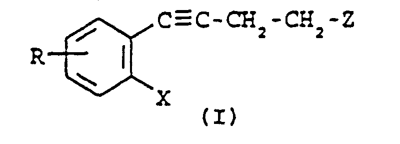 dans laquelle
R est H, CN, F, un groupe formyle, un groupe alkyle en C₁-C₄ facultativement substitué par un ou plusieurs groupes halogéno, alcoxy en C₁-C₃, alkylthio en C₁-C₃, alkylsulfinyle en C₁-C₃ ou alkylsulfonyle en C₁-C₃,
un groupe alcoxy en C₁-C₄ facultativement substitué par un ou plusieurs groupes halogéno, alcoxy en C₁-C₃, alkylthio en C₁-C₃, alkylsulfinyle en C₁-C₃ ou alkylsulfonyle en C₁-C₃,
un groupe alkylthio en C₁-C₄ facultativement substitué par un ou plusieurs groupes halogéno, alcoxy en C₁-C₃, alkylthio en C₁-C₃, alkylsulfinyle en C₁-C₃ ou alkylsulfonyle en C₁-C₃,
un groupe alkylsulfinyle en C₁-C₄ facultativement substitué par un ou plusieurs groupes halogéno, alcoxy en C₁-C₃, alkylthio en C₁-C₃, alkylsulfinyle en C₁-C₃ ou alkylsulfonyle en C₁-C₃,
un groupe alkylsulfonyle en C₁-C₄ facultativement substitué par un ou plusieurs groupes halogéno, alcoxy en C₁-C₃, alkylthio en C₁-C₃, alkylsulfinyle en C₁-C₃ ou alkylsulfonyle en C₁-C₃,
un groupe (alkyle en C₁-C₄)carbonyle facultativement substitué par un ou plusieurs groupes halogéno, alcoxy en C₁-C₃, alkylthio en C₁-C₃, alkylsulfinyle en C₁-C₃ ou alkylsulfonyle en C₁-C₃,
un groupe (alcoxy en C₁-C₄)carbonyle facultativement substitué par un ou plusieurs groupes halogéno ou alcoxy en C₁-C₃,
un groupe di(alkyle en C₁-C₄)amino facultativement substitué par un ou plusieurs groupes halogéno ou alcoxy en C₁-C₃,
un groupe di(alkyle en C₁-C₄)aminocarbonyle facultativement substitué par un ou plusieurs groupes halogéno ou alcoxy en C₁-C₃
un groupe di(alkyle en C₁-C₄)aminosulfonyle facultativement substitué par un ou plusieurs groupes halogéno ou alcoxy en C₁-C₃, ou
un hétérocycle ayant 2 à 6 atomes de carbone et 1 à 3 atomes d'azote, d'oxygène ou de soufre, et qui est facultativement substitué sur les atomes de carbone par un ou plusieurs groupes fluoro, alkyle en C₁-C₄ ou halogénoalkyle en C₁-C₄ ;
X est NO₂ ou NR₁R₂ ;
Z est OH, Br, Cl ou OSO₂R₃ ;
R₁ et R₂ sont chacun indépendamment l'hydrogène ou un groupe formyle, acétyle, halogénoacétyle, CO₂R₄ ou benzyle facultativement substitué sur le noyau aromatique par un à trois groupes fluoro, nitro, alkyle en C₁-C₃ ou alcoxy en C₁-C₃ ; et
R₃ et R₄ sont chacun indépendamment un groupe alkyle en C₁-C₆, halogénoalkyle en C₁-C₆ ou phényle facultativement substitué par un à trois groupes fluoro, nitro, alkyle en C₁-C₃ ou alcoxy en C₁-C₃ ; ou
lorsque X est NR₁R₂, ses sels d'addition d'acide.

2. Composé selon la revendication 1, dans lequel R est H.

3. Composé selon la revendication 2, dans lequel X est NO₂ ou NH₂.

4. Composé selon la revendication 2 ou 3, dans lequel Z est OH.

5. Procédé pour la préparation d'un composé de formule I 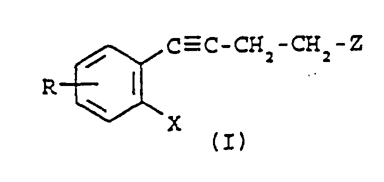 dans laquelle
R est tel que défini dans la revendication 1 ;
X est NO₂ ou NR₁R₂ ;
Z est OH, Br, Cl ou OSO₂R₃ ;
R₁ et R₂ sont chacun indépendamment l'hydrogène ou un groupe formyle, acétyle, halogénoacétyle, CO₂R₄ ou benzyle facultativement substitué sur le noyau aromatique par un à trois groupes fluoro, nitro, alkyle en C₁-C₃ ou alcoxy en C₁-C₃ ; et
R₃ et R₄ sont chacun indépendamment un groupe alkyle en C₁-C₆, halogénoalkyle en C₁-C₆ ou phényle facultativement substitué par un à trois groupes fluoro, nitro, alkyle en C₁-C₃ ou alcoxy en C₁-C₃,
qui consiste à faire réagir un halogénobenzène de formule III 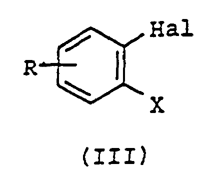 où Hal est Cl, I ou Br et R et X sont tels que définis pour la formule I, avec au moins un équivalent molaire d'un dérivé de butyne de formule IV
HC≡C-CH₂-CH₂-Z (IV)
où Z est tel que défini pour la formule I, en présence d'un quantité à effet catalytique d'un catalyseur au palladium-II et d'un catalyseur au cuivre-I et en présence d'une amine organique.

6. Procédé pour la préparation d'un composé de formule II 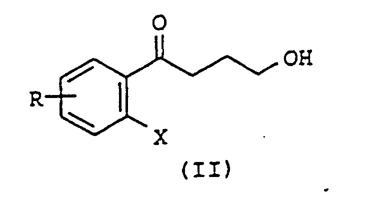 dans laquelle
R est tel que défini dans la revendication 1 ;
X est NO₂ ou NR₁R₂ ;
R₁ et R₂ sont chacun indépendamment l'hydrogène ou un groupe formyle, acétyle, halogénoacétyle, CO₂R₄ ou benzyle facultativement substitué sur le noyau aromatique par un à trois groupes fluoro, nitro, alkyle en C₁-C₃ ou alcoxy en C₁-C₃ ; et
R₄ est un groupe alkyle en C₁-C₆, halogénoalkyle en C₁-C₆ ou phényle facultativement substitué par un à trois groupes fluoro, nitro, alkyle en C₁-C₃ ou alcoxy en C₁-C₃,
qui consiste à faire réagir un composé de formule III 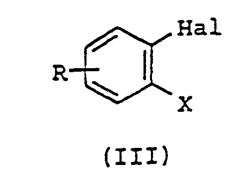 où X et R sont tels que définis ci-dessus et Hal est Cl, I ou Br, avec au moins un équivalent molaire de 3-butyne-1-ol en présence d'une quantité à effet catalytique d'un catalyseur au palladium-II et d'un catalyseur au cuivre-I et en présence d'une amine organique pour obtenir un composé intermédiaire de formule Ia 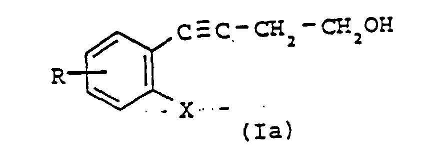 où R et X sont tels que définis ci-dessus, et à hydrater le composé intermédiaire de formule Ia en présence d'eau et d'un agent d'hydratation pour obtenir le composé désiré de formule II.

7. Procédé pour la préparation d'un composé de formule VII 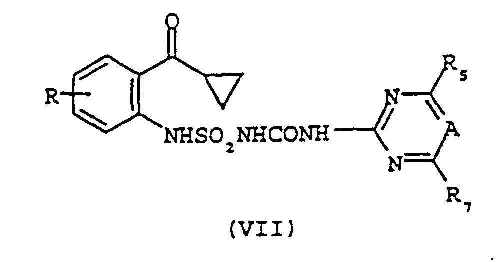 dans laquelle
R est tel que défini dans la revendication 1 et
A est N ou CR₆ ;
R₅ est H, un halogène, un groupe alkyle en C₁-C₄ facultativement substitué par un ou plusieurs groupes halogéno ou alcoxy en C₁-C₃,
un groupe alcoxy en C₁-C₄ facultativement substitué par un ou plusieurs groupes halogéno ou alcoxy en C₁-C₃,
un groupe alkylthio en C₁-C₄ facultativement substitué par un ou plusieurs groupes halogéno ou alcoxy en C₁-C₃,
un groupe alkylsulfinyle en C₁-C₄ facultativement substitué par un ou plusieurs groupes halogéno ou alcoxy en C₁-C₃,
un groupe alkylsulfonyle en C₁-C₄ facultativement substitué par un ou plusieurs groupes halogéno ou alcoxy en C₁-C₃, ou
un groupe alkylamino en C₁-C₄ ou di(alkyle en C₁-C₄)amino, chaque groupe alkyle étant facultativement substitué par un ou plusieurs groupes halogéno ou alcoxy en C₁-C₃ ;
R₆ est H ou un halogène ; et
R₇ est l'hydrogène, un groupe alkyle en C₁-C₄ facultativement substitué par un ou plusieurs groupes halogéno ou alcoxy en C₁-C₃,
un groupe alcoxy en C₁-C₄ facultativement substitué par un ou plusieurs groupes halogéno ou alcoxy en C₁-C₃,
un groupe alkylthio en C₁-C₄ facultativement substitué par un ou plusieurs groupes halogéno ou alcoxy en C₁-C₃,
un groupe alkylsulfinyle en C₁-C₄ facultativement substitué par un ou plusieurs groupes halogéno ou alcoxy en C₁-C₃,
un groupe alkylsulfonyle en C₁-C₄ facultativement substitué par un ou plusieurs groupes halogéno ou alcoxy en C₁-C₃, ou
un groupe alkylamino en C₁-C₄ ou di(alkyle en C₁-C₄)amino, chaque groupe alkyle étant facultativement substitué par un ou plusieurs groupes halogéno ou alcoxy en C₁-C₃,
qui comprend les étapes suivantes :
a) faire réagir un halogénobenzène de formule III 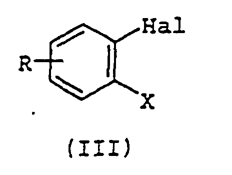 où R est tel que défini dans la revendication 1 ;
X est NO₂ ou NH₂, et Hal est Cl, I ou Br, avec au moins un équivalent molaire de 3-butyne-1-ol en présence d'une quantité à effet catalytique d'un catalyseur au palladium-II et d'un catalyseur au cuivre-I et en présence d'une amine organique pour former un aryne de formule Ia 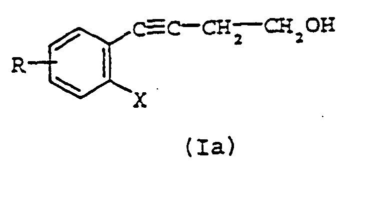 où R et X sont tels que définis ci-dessus ;
b) hydrater ledit aryne de formule I avec un agent d'hydratation pour obtenir une 4-hydroxy-(*o*-nitrophényl)butanone ou une 4-hydroxy-(*o*-aminophényl)butanone de formule II 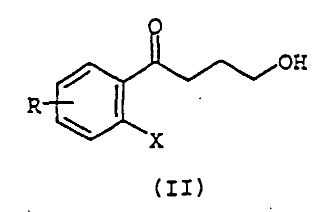 où R et X sont tels que définis ci-dessus ;
c) halogéner ladite 4-hydroxybutanone avec un halogénure d'hydrogène pour obtenir une 4-halogénobutanone de formule V 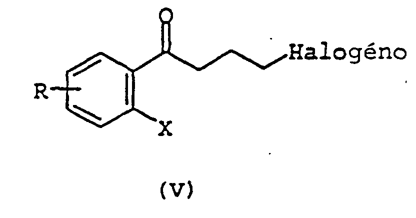 où R est tel que défini ci-dessus ;
X est NO₂ ou NH₂, et Halo est Cl, Br, I ou F ;
d) déshydrohalogéner ledit composé de formule V en présence d'une base et d'un catalyseur de transfert entre phases et facultativement en présence d'un solvant pour former une *o*-nitrophényl-cyclopropyl-cétone ou une *o*-aminophényl-cyclopropyl-cétone de formule VI 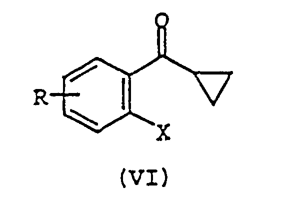 où R est tel que défini ci-dessus et X est NH₂ ou NO₂ ;
e) lorsque X est NO₂, réduire ladite o-nitrophényl-cyclopropyl-cétone de formule VI en présence d'hydrogène et d'un catalyseur, facultativement en présence d'un solvant, pour former la *o*-aminophényl-cyclopropyl-cétone correspondante de formule VI dans laquelle X est NH₂ ; et
f) faire réagir ladite *o*-aminophényl-cyclopropyl-cétone de formule VI avec un composé 2-aminoarylique de formule VIII 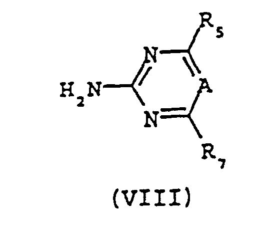 où A, R₅ et R₇ sont tels que définis ci-dessus,
et de l'isocyanate de chlorosulfonyle en présence de triéthylamine et d'un solvant pour obtenir la sulfamoylurée désirée de formule VII.

8. Procédé selon la revendication 5, 6 ou 7, dans lequel le catalyseur au palladium-II est le chlorure de bis(triphénylphosphine)-palladium-II et le catalyseur au cuivre-I est l'iodure de cuivre-I ; l'amine organique est la triéthylamine ou la diéthylamine ; le procédé utilise un composé de formule III dans lequel R est H et X est NO₂ ou NH₂.

9. Procédé selon la revendication 6 ou 7, dans lequel l'agent d'hydratation est HgO, H₂SO₄ ou Na₂S, HCl.

10. Procédé selon la revendication 7, pour préparer un composé de formule VII dans lequel A est CR₆ ; R et R₆ sont chacun H ; et R₅ et R₇ sont chacun un groupe méthoxy.

11. Procédé pour la préparation d'un composé ayant la formule VII telle que définie et représentée dans la revendication 7, qui consiste à
(a) hydrater un composé ayant la formule I telle que définie et représentée dans la revendication 1 avec un agent d'hydratation et, si Z est -OH, faire réagir le produit d'hydratation avec un réactif pour échanger le groupe hydroxyle contre du chlore ou du brome ou avec un dérivé réactif d'un acide sulfonique ayant la formule R₃SO₂OH où R₃ est tel que défini dans la revendication 1 afin de convertir le groupe OH représenté par Z en Br, Cl ou OSO₂R₃ où R₃ est tel que défini ci-dessus ;
(b) cycliser le composé résultant ayant la formule IX 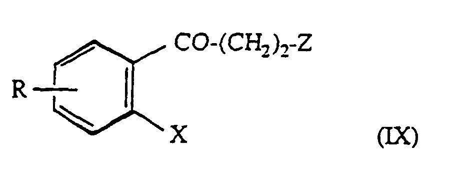 où R et X sont tels que définis dans la revendication 1 et Z est Br, Cl ou OSO₂R₃ où R₃ est tel que défini dans la revendication 1, afin de former un groupe cyclopropylcarbonyle à la position *ortho par* rapport à X et, si X est autre chose que -NH₂ dans le produit obtenu, convertir X en -NH₂ par réduction ou hydrolyse ; et
(c) faire réagir le composé résultant ayant la formule VI telle que représentée dans la revendication 7 où X est NH₂ et R est tel que défini dans la revendication 1 avec un réactif formateur de sulfonamide qui introduit un groupe ayant la formule X 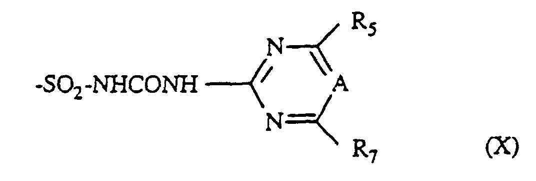 où A, R₅ et R₇ sont tels que définis dans la revendication 7.
